(19) Europäisches Patentamt

European Patent Office

Office européen des brevets

(11) Publication number: **0 409 490 A1**

# EUROPEAN PATENT APPLICATION

(21) Application number: 90307634.7

(22) Date of filing: 12.07.90

(51) Int. Cl.5: **A61K 31/70**, //(A61K31/70, 31:195,31:19,31:045),A61K47:10

(30) Priority: 18.07.89 GB 8916394

(43) Date of publication of application: 23.01.91 Bulletin 91/04

(84) Designated Contracting States: CH DE FR GB IT LI NL

(71) Applicant: Beecham Group p.l.c.
SB House Great West Road
Brentford Middlesex TW8 9BD(GB)

(72) Inventor: Bywater, Robert James, SmithKline Beecham
Pharmaceuticals, Walton Oaks, Dorking Road
Tadworth, Surrey KT20 7NT(GB)
Inventor: Dupe, Robert John, SmithKline Beecham
Pharmaceuticals, Walton Oaks, Dorking Road
Tadworth, Surrey KT20 7NT(GB)

(74) Representative: West, Vivien et al
SmithKline Beecham, Corporate Patents,
Great Burgh, Yew Tree Bottom Road
Epsom, Surrey KT18 5XQ(GB)

(54) **Method of treatment.**

(57) A veterinary composition for the treatment of conditions associated with low plasma glucose levels including pregnancy toxaemia in ewes and bovine ketosis, comprises an actively absorbed mono-saccharide, an actively absorbed naturally occurring amino acid, an optionally substituted $C_{1-6}$ alkyl carboxylic acid and/or a salt thereof, and a polyhydric alcohol.

EP 0 409 490 A1

## METHOD OF TREATMENT

This invention relates to a method of treating disorders in domestic animals, and in particular to a method of treating disorders associated with low plasma glucose levels, for example pregnancy toxaemia in ewes and bovine ketosis in lactating cows.

Veterinary compositions which are suitable for treating animals suffering from conditions associated with low plasma glucose levels are known. For example a product having the trade name Ketol, available from Intervet Lab. Ltd, has been widely used in cases of ovine pregnancy toxaemia and is formulated from propylene glycol, choline chloride and trace amounts of potassium iodide.

In addition, European Patent Application Publication No. 0 226 332 describes a veterinary composition used for rehydrating domestic animals, which is also stated to be of value in the treatment of hypo-glycaemia associated with ovine or bovine ketosis.

A combination of substances has now been found which is particularly effective in increasing plasma glucose concentrations when administered to domestic animals.

Accordingly, the present invention provides a method for the treatment of conditions associated with low plasma glucose levels, including pregnancy toxaemia in ewes and bovine ketosis, which comprises the administration to an animal in need thereof of an actively absorbed mono-saccharide, an actively absorbed naturally occurring amino acid, an optionally substituted $C_{1-6}$ alkyl carboxylic acid and/or a salt thereof, and a polyhydric alcohol.

The above combination of substances may be administered simultaneously or sequentially. A particular aspect of the invention provides a veterinary product for use in carrying out the method of the invention, which product comprises an actively absorbed mono-saccharide, an actively absorbed naturally occurring amino acid, an optionally substituted $C_{1-6}$ alkyl carboxylic acid and/or a salt thereof, and a polyhydric alcohol. More especially the product consists of a first component comprising the mono-saccharide, the amino acid, and the carboxylic acid and/or salt thereof, and a second component comprising or consisting essentially of the polyhydric alcohol. Typically the first and second components are contained in first and second containers respectively.

The first component is preferably an aqueous composition or alternatively is provided as a dry powder composition to be made up with water prior to administration to the animal. The ratio of aqueous composition: second component to be administered to the animal is typically in the range 7:3 to 8:2, more preferably 7.2:2.8 to 7.6:2.4, v/v. The first and second components may be administered simultaneously or sequentially; for example they may be mixed immediately before administration to the animal.

Thus, in a particular aspect of the invention the product comprises an aqueous solution containing 200ml to 300ml, preferably 240ml to 280ml, of polyhydric alcohol per litre. Advantageously, the polyhydric alcohol is propylene glycol.

As used herein "actively absorbed" has the meaning defined in EP-A-0 226 332.

Advantageously, the mono-saccharide is glucose, for example, dextrose, and comprises 10 to 35% w/v of the product (corresponding to 12 to 50% w/v of the first component when present).

Suitably, the amino acid is glycine, and comprises 0.5 to 12% w/v of the product (corresponding to 0.6 to 17% w/v of the first component when present).

An example of an optionally substituted $C_{1-6}$ alkyl carboxylic acid and/or a salt thereof is citric acid and the sodium and potassium salts (including acid salts) thereof. Typically the total citrate content of the product is in the range 0.1 to 4% w/v (corresponding to 0.12 to 5.7% w/v of the first component when present).

Typically the product will also contain 1 to 10% w/v, preferably 1 to 8% w/v, electrolytes such as sodium chloride (corresponding to 1.2 to 14% w/v, preferably 1.2 to 11.4% w/v, of the first component when present).

Preferably the product will also comprise a buffering agent such as potassium dihydrogen phosphate, typically in the range of 0.1 to 2.5 %w/v (corresponding to 0.12 to 3% w/v of the first component when present). Suitably the product is formulated to have a final pH of 3 to 6, preferably about 4.0.

The product may contain further conventional ingredients such as preservatives and colouring agents.

A further aspect of the invention provides a process for the preparation of a product of the invention which comprises mixing the ingredients thereof in the necessary proportions.

The product may be administered orally for example by drenching or via stomach tube.

In general ewes will have administered about 200ml to 300ml per dose while lactating cows will normally be administered about 1000ml per dose. Dosing can be repeated if required, at 4 to 6 hourly intervals.

The method of the invention has been found to enhance plasma glucose levels and reduce ketone levels in toxaemic sheep.

The following Example illustrates the invention.

Example 1

A total of 160 ml of the following composition was prepared by mixing together the following ingredients:-

|  | Grams |
| --- | --- |
| Dextrose monohydrate | 44.6g |
| Glycine | 6.17g |
| Sodium chloride | 8.55g |
| Potassium dihydrogen phosphate | 4.084g |
| Citric acid monohydrate | 0.525g |
| Tri-potassium citrate monohydrate | 0.12g |
| Sodium metabisulphite | 0.275g |
| Deionized water | to 160 ml |

The composition was filled into a first container, and 60ml of propylene glycol was filled into a second container.

CLINICAL DATA

1. The effect on the plasma glucose concentrations in lactacting cows was examined in a cross-over study using the composition of Example 1 of the present invention (LC + PG) and one without propylene glycol (LC).

Method

Two groups of three cows were dosed by drenching with a beer bottle. The cows were milked before dosing commenced. Initially three cows were drenched with 760ml LC, four days later a further three cows were given 760ml LC + 240ml PG, these treatments were crossed over during the following two days.

The cows were not allowed food on the morning of the dosing, nor during the sampling period.

All samples were taken by jugular venepuncture into 5ml vacutainers containing potassium oxalate/sodium fluoride to prevent glucose breakdown. Blood samples were taken at 0, 0.25, 0.50, 0.75, 1.0, 1.5, 2.0, 4.0, 6.0 and 8.0 hrs.

Results

Results expressed as plasma glucose concentrations (Table 1) show that LC + PG gave an elevated and extended plasma glucose peak compared to LC alone.

3

Table 1

| Plasma glucose concentrations (mM/C) in cows dosed with LC | | | | | | | | | | | |
|---|---|---|---|---|---|---|---|---|---|---|---|
| Cow No. | Sample time in hours after dosing | | | | | | | | | | |
| | 0 | 0.25 | 0.50 | 0.75 | 1 | 1.5 | 2 | 4 | 6 | 8 | 24 |
| 703 | 3.36 | 3.79 | 3.60 | 3.87 | 4.47 | 4.08 | 3.80 | 3.89 | 3.56 | 3.71 | 3.68 |
| 173 | 3.51 | 3.43 | 3.30 | 3.44 | 3.75 | 3.21 | 3.17 | 3.20 | 3.31 | 3.37 | 3.24 |
| 81 | 3.38 | 3.51 | 3.61 | 3.45 | 3.46 | 3.42 | 3.40 | 3.49 | 3.38 | 3.60 | 2.97 |
| 75 | 3.19 | 3.59 | 3.61 | 3.87 | 4.12 | 4.00 | 3.62 | 3.34 | 3.44 | 3.58 | 3.22 |
| 133 | 3.40 | 3.73 | 4.44 | 4.00 | 4.65 | 4.69 | 4.08 | 3.54 | 3.74 | 3.55 | 3.31 |
| 76 | 3.50 | 3.43 | 3.98 | 4.34 | 4.47 | 4.09 | 3.81 | 3.59 | 3.44 | 3.42 | 3.26 |
| Mean | 3.39 | 3.58 | 3.76 | 3.83 | 4.15 | 3.92 | 3.65 | 3.51 | 3.48 | 3.54 | 3.28 |
| ± SD | 0.12 | 0.15 | 0.40 | 0.34 | 0.47 | 0.53 | 0.32 | 0.24 | 0.15 | 0.12 | 0.23 |
| Plasma glucose concentrations (mM/C) in cows dosed with LC + PG | | | | | | | | | | | |
| Cow No. | Sample time in hours after dosing | | | | | | | | | | |
| 703 | 3.35 | 3.85 | 4.62 | 4.64 | 5.16 | 5.30 | 4.53 | 3.66 | 3.74 | 4.07 | 3.60 |
| 173 | 2.99 | 3.54 | 3.70 | 3.53 | 3.72 | 3.64 | 3.83 | 3.33 | 3.50 | 3.45 | 3.55 |
| 81 | 3.24 | 3.75 | 4.05 | 4.14 | 4.26 | 4.03 | 3.84 | 3.55 | 3.77 | 3.75 | 3.63 |
| 75 | 3.52 | 3.82 | 4.17 | 4.52 | 5.05 | 5.12 | 4.71 | 3.35 | 3.76 | 3.61 | 3.58 |
| 133 | 3.47 | 3.92 | 4.53 | 4.81 | 5.79 | 5.95 | 6.06 | 3.86 | 3.76 | 3.84 | 3.56 |
| 76 | 3.23 | 3.60 | 4.07 | 4.47 | 4.66 | 4.95 | 4.69 | 3.35 | 3.51 | 3.59 | 2.98 |
| Mean | 3.30 | 3.75 | 4.19 | 4.35 | 4.77 | 4.83 | 4.61 | 3.52 | 3.67 | 3.72 | 3.48 |
| ± SD | 0.19 | 0.15 | 0.34 | 0.46 | 0.73 | 0.85 | 0.82 | 0.21 | 0.13 | 0.22 | 0.25 |

2. The effect on the plasma glucose concentrations in healthy ewes was examined in a cross-over study using the composition of Example 1 of the present invention (LC + PG) and one without propylene glycol (LC).

Method

Six healthy non-pregnant ewes were selected, weighed and fasted on the day prior to dosing but were allowed water ad-libitum .

The same 6 ewes were used on each occasion, separated by 10 days.

On the first occasion, all dosing was by stomach tube (i.e., intrarumenally). Three of the ewes were given 160ml LC, the other three were given 160ml LC + 60ml PG. These treatments were crossed over after a day.

The second occasion was a treatment regime repeat, but all dosing was performed by drench bottle. On this occasion, the bottle alone was used without its catheter, to encourage voluntary swallowing and to deliver the dose into the abomasum, bypassing the rumen.

Ewes were fasted for 20 hours prior to base line blood samples being taken. All samples were taken from the jugular vein into 5ml vacutainers containing potassium oxalate/sodium fluoride to prevent glucose breakdown. Dosing samples were taken at 15, 30, 45, 60, 120, 180, 240, 360, 480 and 1440 mins. Storage where necessary, was at 4°C until the samples could be centrifuged (10 min./3500 rpm), decanted in fluoride containers and sent for glucose analysis (Automated Hexokinase).

Results

The results from the first part of the trial, where the ewes were stomach tubed, showed that plasma glucose levels were low (Table 2), this was probably a consequence of dosing into the rumen (by-passing the oesophageal groove) where the absorption rate would be much slower.

The second part of the trial was conducted by drenching, where the closure of the oesaphageal groove may take place (Table 3).

The results of both trials showed that LC + PG gave an elevated and extended peak, although this was much more prominent when the solution was stomach tubed into the rumen.

Table 2

| Mean plasma glucose concentrations in ewes dosed (stomach tubed) with LC and LC + PG | | | | | | |
|---|---|---|---|---|---|---|
| Sample Time | LC conc. mMoles/l | | | LC + propylene glycol mMoles/l | | |
| | Mean | SD | SEM | Mean | SD | SEM |
| 0 | 2.87 | 0.16 | 0.07 | 2.87 | 0.45 | 0.19 |
| 15 | 3.67 | 0.45 | 0.18 | 3.72 | 0.83 | 0.34 |
| 30 | 3.81 | 0.90 | 0.37 | 3.97 | 0.84 | 0.34 |
| 45 | 3.91 | 0.89 | 0.37 | 4.14 | 0.93 | 0.38 |
| 1 | 3.99 | 1.28 | 0.52 | 4.24 | 0.97 | 0.39 |
| $1\frac{1}{2}$ | 3.95 | 1.04 | 0.42 | 2.49 | 1.35 | 0.50 |
| 2 | 3.68 | 0.78 | 0.32 | 4.35 | 1.34 | 0.50 |
| 3 | 3.32 | 0.20 | 0.08 | 4.31 | 1.36 | 0.50 |
| 4 | 3.20 | 0.26 | 0.11 | 4.19 | 1.02 | 0.40 |
| 6 | 3.12 | 0.26 | 0.10 | 3.86 | 0.60 | 0.25 |
| 8 | 3.10 | 0.14 | 0.05 | 3.50 | 0.50 | 0.19 |
| 24 | 2.84 | 0.16 | 0.05 | 3.08 | 0.17 | 0.07 |

Table 3

| Mean plasma glucose concentrations in ewes dosed (drenched) with LC and LC + PG | | | | | | |
|---|---|---|---|---|---|---|
| Sample Time | LC conc. mMoles/l | | | LC + propylene glycol mMoles/l | | |
| | Mean | SD | SEM | Mean | SD | SEM |
| 0 | 2.91 | 0.15 | 0.06 | 2.95 | 0.24 | 0.10 |
| 15 | 3.99 | 0.51 | 0.21 | 3.81 | 0.50 | 0.20 |
| 30 | 4.82 | 1.03 | 0.42 | 4.56 | 1.00 | 0.41 |
| 45 | 5.15 | 1.26 | 0.52 | 5.10 | 1.31 | 0.53 |
| 1 | 5.23 | 1.36 | 0.56 | 5.32 | 1.63 | 0.73 |
| $1\frac{1}{2}$ | 4.78 | 1.01 | 0.41 | 4.98 | 1.31 | 0.53 |
| 2 | 4.59 | 1.01 | 0.41 | 4.61 | 1.01 | 0.41 |
| 3 | 3.52 | 0.33 | 0.13 | 3.95 | 0.50 | 0.20 |
| 4 | 3.26 | 0.38 | 0.15 | 3.44 | 0.48 | 0.20 |
| 6 | 2.89 | 0.19 | 0.08 | 3.41 | 0.72 | 0.30 |
| 8 | 3.03 | 0.19 | 0.08 | 3.44 | 0.50 | 0.20 |
| 24 | 2.96 | 0.17 | 0.07 | 3.00 | 0.14 | 0.06 |

## Claims

1. A veterinary product comprising an actively absorbed mono-saccharide, an actively absorbed naturally occurring amino acid, an optionally substituted $C_{1-6}$ alkyl carboxylic acid and/or a salt thereof, and a polyhydric alcohol.

2. A product according to claim 1, which consists of a first component comprising the mono-saccharide, the amino acid and the carboxylic acid and/or a salt thereof, and a second component comprising or consisting essentially of the polyhydric alcohol.

3. A product according to claim 2, wherein the first component comprises an aqueous composition or is a dry powder composition.

4. A product according to claim 3, wherein the ratio of aqueous composition: second component is 7:3 to 8:2 v/v.

5. A product according to any one of claims 1 to 4, comprising an aqueous solution containing 200 to 300ml of polyhydric alcohol per litre and wherein the polyhydric alcohol is propylene glycol.

6. A product according to any one of the preceding claims wherein the mono-saccharide is dextrose and comprises 10 to 35% w/v of the product.

7. A product according to any one of the preceding claims wherein the amino acid is glycine and comprises 0.5 to 12% w/v of the product.

8. A p.roduct according to any one of the preceding claims wherein the optionally substituted $C_{1-6}$ alkyl carboxylic acid and/or a salt thereof is citric acid and/or a sodium or potassium salt thereof, and the total citrate content of the product is in the range 0.1 to 4% w/v.

9. A product according to any one of the preceding claims, for use in the treatment of conditions associated with low plasma glucose levels.

10. A process for the preparation of a veterinary product as claimed in any one of claims 1 to 8, which process comprises mixing the ingredients thereof in the necessary proportions.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 226 332 (BEECHAM GROUP PLC) --- | 1-10 | A 61 K 31/70 // A 61 K 47/10 (A 61 K 31/70 A 61 K 31:195 A 61 K 31:19 A 61 K 31:045) |
| A | EP-A-0 190 459 (PFRIMMER + CO.) --- | 1-10 | |
| A | EP-A-0 312 249 (TAKEDA CHEMICAL INDUSTRIES, LTD) ----- | 1-10 | |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 03-10-1990 | BRINKMANN C. |

EPO FORM 1503 03.82 (P0401)